(19) Europäisches Patentamt

European Patent Office

Office européen des brevets

(11) **EP 1 075 836 A2**

(12) **EUROPEAN PATENT APPLICATION**

(43) Date of publication:
14.02.2001 Bulletin 2001/07

(21) Application number: 00303523.5

(22) Date of filing: 27.04.2000

(51) Int. Cl.$^7$: **A61K 7/48**, A23G 3/00, A23G 3/30, A23L 1/06, A23L 1/40, A23L 2/52, A21D 2/18

(84) Designated Contracting States:
**AT BE CH CY DE DK ES FI FR GB GR IE IT LI LU MC NL PT SE**
Designated Extension States:
**AL LT LV MK RO SI**

(30) Priority: **09.08.1999 JP 22524599**

(71) Applicant:
**Yaizu Suisankagaku Industry Co., Ltd.
Yaizu-shi, Shizuoka-ken (JP)**

(72) Inventors:
• **Matahira, Yoshiharu
Shimada-shi, Shizuoka-ken (JP)**
• **Saito, Michiko
Osuka-cho, Shizuoka-ken (JP)**

(74) Representative:
**Towler, Philip Dean
Frank B. Dehn & Co.,
European Patent Attorneys,
179 Queen Victoria Street
London EC4V 4EL (GB)**

(54) **Skin care of food composition containing N-acetyl-glucosamine**

(57) The present invention provides a skin care agent comprising N-acetylglucosamine as an active ingredient. The skin care agent is preferably in the form of tablets, capsules, powder such as dust or granules, liquid or paste. The skin care agent of the present invention may be incorporated into foods such as confectioneries, powdered soup and beverages. By orally ingesting the skin care agent of the present invention, the N-acetylglucosamine as an active ingredient is rapidly absorbed, and by utilizing a part thereof as a starting material of acidic mucopolysaccharides such as hyaluronic acid or chondroitin sulfate, the moisture and tension of skin can be improved and the rough skin and fine wrinkles can be prevented or ameliorated.

EP 1 075 836 A2

**Description**

Field of the Invention

The present invention relates to a skin care agent (or a skin-beautification promotor) which improves moisture and tension of skin and promotes prevention and amelioration of e.g. rough skin and fine wrinkles by orally ingesting it, and a health and beauty care food containing it.

Background of the Invention

Acidic mucopolysaccharides such as hyaluronic acid or chondroitin sulfate have a high water retention, bond to collagen which serves as a columnar intercellular matrix substance and are mostly distributed in, for example , connective tissues, cartilaginous tissues and skin tissues, thereby being useful for keeping functions and forms of cells.

In the skin tissues, the acidic mucopolysaccharides, collagen, etc. mostly exist in the corium layer and play a large part in water retention and resilience of skin. It is known that when the amounts thereof decrease due to aging or the like, the water retention and resilience of skin will be lost, thereby causing rough skin, fine wrinkles, etc.

Accordingly, in order to prevent and ameliorate the rough skin and fine wrinkles, it is important to maintain the moisture and tension of skin. For this purpose, cosmetics in which various components having effects for maintaining the moisture retention and resilience of skin are incorporated, are commercially available. As examples of such components, the mucopolysaccharides such as hyaluronic acid, chondroitin sulfate and collagen, low molecular weight saccharides such as trehalose and sorbitol, vitamins, amino acid derivatives, ceramide, α-orizanol, and fats and oils such as refined camellia oil, may be mentioned. In particular, due to recent developments, highly safe components derived from natural substances are likely to be regarded as more worthy.

Furthermore, many health and beauty care foods have been developed which enhance the above-mentioned effects by oral ingestion. For example, health and beauty care foods comprising nucleic acids and mucopolysaccharides which contain hyaluronic acid, chondroitin sulfate and collagen (Japanese Unexamined Patent Publication No. 10-165138), processed foods comprising as a main component a mixture of at least two food materials of active oxygen elimination factors, antiallergic factors, factors for improving e.g. skin, and antioxidation factors (Japanese Unexamined Patent Publication No. 10-70), foods comprising conchiolin or its processed product (Japanese Unexamined Patent Publication No. 8-173091), health foods comprising conjugated mucopolysaccharide wherein a mucopolysaccharide and peptide are bonded (Japanese Unexamined Patent Publication No. 9-98739), and health foods containing ceramide (Japanese Unexamined Patent Publication No. 11-113530), may be mentioned.

On the other hand, N-acetylglucosamine is one of the natural aminosugars obtainable by decomposing a high molecular weight polysaccharide chitin contained in shells of crustacea such as crab and shrimp or lobster, and is a white crystalline powder having a good sweetness of about half that of sugar absorbing less moisture. N-acetylglucosamine is also contained in milk in a free state in an amount of about 10 mg/100 ml, and exists universally in living organisms as constituting units of sugar chains of mucopolysaccharide, glycoprotein and glucolipide. N-acetylglucosamine is usually produced from glucose as a starting material by metabolism in living organisms, and is one of the components of living organisms which is highly safe for human beings. As physiological actions of N-acetylglucosamine, amelioration of arthritis symptoms, propagation-accelerating effect of Lactobacillus vifidus, and the like, have been known.

However, since hyaluronic acid, chondroitin sulfate, collagen and the like are high molecular weight compounds and are hardly absorbed unless coated on the skin like cosmetics, these components are used for the purpose of improving the water retention of the skin surface when used as cosmetics. Furthermore, this is true for most of the above-mentioned other components. Furthermore, if the high molecular weight compounds such as hyaluronic acid, chondroitin sulfate and collagen, are orally ingested, there are problems with digestion and absorption and their effects are not necessarily satisfactory.

Summary of the Invention

Accordingly, it is an object of the present invention to provide a skin care agent and a health and beauty care food, which has an action such as the improvement of moisture and tension of skin and prevention and amelioration of e.g. rough skin and fine wrinkles by oral ingestion.

The present inventors have intensively studied how to accomplish the above object, and as a result, have found that N-acetylglucosamine orally ingested is rapidly absorbed from the intestine and reaches the cutaneous layer, and in the cutaneous layer, promotes biosynthesis of mucopolysaccharides such as hyaluronic acid; and have accomplished the present invention.

Namely, the present invention provides a skin care agent comprising N-acetylglucosamine as a main component.

According to the present invention wherein N-acetylglucosamine is incorporated into the skin care agent. When it is ingested, most of the N-acetylglucosamine is rapidly absorbed and a part thereof is utilized as a starting material of mucopolysaccharides such as hyaluronic acid or chondroitin sulfate, whereby the moisture and tension of skin can be improved and the rough skin and fine wrinkles can be prevented and ameliorated.

Brief Description of the Drawings

Fig.1 is a photograph showing the results of autoradiography of rat to which radioactivity-labelled N-acetylglucosamine is administered.
Fig.2 is a graph showing the tissue distribution of radioactivity after administration of radioactivity-labelled N-acetylglucosamine.

Description of the Preferred Embodiments

Hereinafter, the present invention will be described in further detail with reference to preferred embodiments.
In the present invention, as N-acetylglucosamine (hereinafter referred to as "NAG"), NAG obtainable by hydrolysis of natural polysaccharide chitins derived from shells of crustacea such as crab and shrimp or lobster with an acid or an enzyme (natural-type NAG), or NAG obtainable by acetylation by chemical synthesis of a D-glucosamine chlorate which is obtainable by complete acidic hydrolysis of chitin (chemically synthesized NAG), may be used.
For example, the above-mentioned natural-type NAG can be obtained by methods disclosed in Japanese Patent No. 1822027, etc.
In the present invention, NAG is contained as an active ingredient in the skin care agent in an amount of preferably from 0.1 to 100 % by weight, more preferably from 1 to 50 % by weight.
According to more preferred embodiments of the present invention, the skin care agent should preferably contain other components which have already been recognized to have a skin-beautification (or skin care) effect, for example, collagen, chondroitin sulfate, hyaluronic acid, vitamin C, vitamin B group, trehalose and ceramide. Among these, it is preferred that the skin care agent contains at least one component selected from collagen, chondroitin sulfate and vitamin C. In this instance, as the proportional ratios of respective components, it is further preferred that collagen is from 1 to 20 parts by weight, chondroitin sulfate is from 0.5 to 10 parts by weight and vitamin C is from 0.1 to 10 parts by weight, based on 1 part of NAG by weight.
According to this embodiment, since NAG and the other component(s) having the skin-beautification effect can be ingested together, synergistic effects of skin-beautification (or skin care) promotion can be expected. Further, even if the above other components co-exist, NAG causes no reaction such as coloring or decomposition.
The uptake of the skin care agent of the present invention is preferably from 0.1 to 15 g, more preferably from 0.3 to 5 g in terms of NAG per day for adult. If the uptake of NAG is less than 0.1 g, the skin-beautification effect cannot be expected, and this is undesirable. Furthermore, if the uptake of NAG exceeds 15 g, there is a possibility of symptoms such as pasty stools or diarrhoea, and this is undesirable. In this regard, as shown in the test examples as described below, safety is confirmed even if NAG is orally administered in an amount of 5 g per kg of body weight of rat.
The skin care agent of the present invention preferably takes the form of tablets, capsules, powder, granules, liquid or paste.
For example, the tablets are obtained by uniformly mixing the components having the skin-beautification effect together with excipients, and tabletting the mixture with a pressure-type tabletting machine.
The powder and granules are obtainable by using the above mixture as it is or subjecting it to granulation.
The capsules are obtainable by uniformly dispersing NAG and the other components having the skin-beautification effect in fats and oils such as safflower oil, and then adding e.g. beeswax thereto to appropriately adjust the viscosity of the slurry, followed by filling it into a soft capsule made of gelatin and glycerol as the main components of encapsulating materials with a soft capsule filling machine.
Furthermore, NAG has a solubility of 32% by weight in water at 25°C, is confirmed not to show coloration or decomposition even if heat treated at a pH of 2 to 8 at 100°C for 1 hour, and has a stability in usual food processing without any problem at all, so that the skin care agent of the present invention can be added to foods such as confectioneries, powdered soups, dairy products and beverages. As such foods, specifically, gum, candies, tabletted confectioneries, chocolate, jelly, cookies, snacks, corn potage soup, consomme soup, milk, pudding, yogurt, ice cream, lactic acid beverages, alcohol beverages, vitamin beverages, mineral beverages, coffee beverages, mineral water, nutrition drinks, and the like, may be mentioned.
When the skin care agent of the present invention is added to foods, the added amount is preferably from 0.01 to 100 % by weight, more preferably from 1 to 50 % by weight. Further, NAG should preferably be contained in an amount of from 0.1 to 15 g, more preferably from 0.3 to 5 g, per meal.

PREPARATION EXAMPLE 1 (Preparation of NAG)

4 kg of chitin was added to 12 liters of conc. hydrochloric acid and partial hydrolysis was carried out while stirring at 40°C for 3 hours. After the completion of the hydrolysis, the resultant mixture was diluted with water of the same volume, and neutralized to pH 5.0 with a 25% sodium hydroxide solution. 500 g of activated carbon was added to this neutralized solution, and stirring was carried out for 30 minutes to decolorize, and then filtration was carried out with a filter paper and insolubles and the activated carbon were removed to obtain 42 liters of a filtrate. This filtrate was subjected to deionization by an ion exchange membrane electrodialyser to obtain about 20 liters of the deionized liquid. This deionized liquid contained about 1.7 kg of chitinoligosaccharide. To this chitinoligosaccharide-containing solution, 50,000 units of chitinase (manufactured by Sigma Co.) was added, and then an enzyme was permitted to react therewith at 45°C for 50 hours to decompose the chitinoligosaccharide and form NAG. After deactivation of the enzyme by heating, undecomposed chitinoligosaccharide was removed by treatment with 1 kg of activated carbon, and then treatment with an ion exchange resin was carried out, followed by condensation and freeze drying to obtain 1.35 kg of NAG having a purity of 99.5%.

TEST EXAMPLE 1 (Acute toxic test of NAG)

To each of five male and five female Wister rats (SPF), NAG was given by a single oral administration in an amount of 5,000 mg per kg of body weight. After the administration, these rats were bred for 14 days and observed, and no death was recognized. It was also recognized that the 50 % lethal dose ($LD_{50}$) to the rats was at least 5,000 mg per kg of body weight.

TEST EXAMPLE 2 (NAG kinetics study)

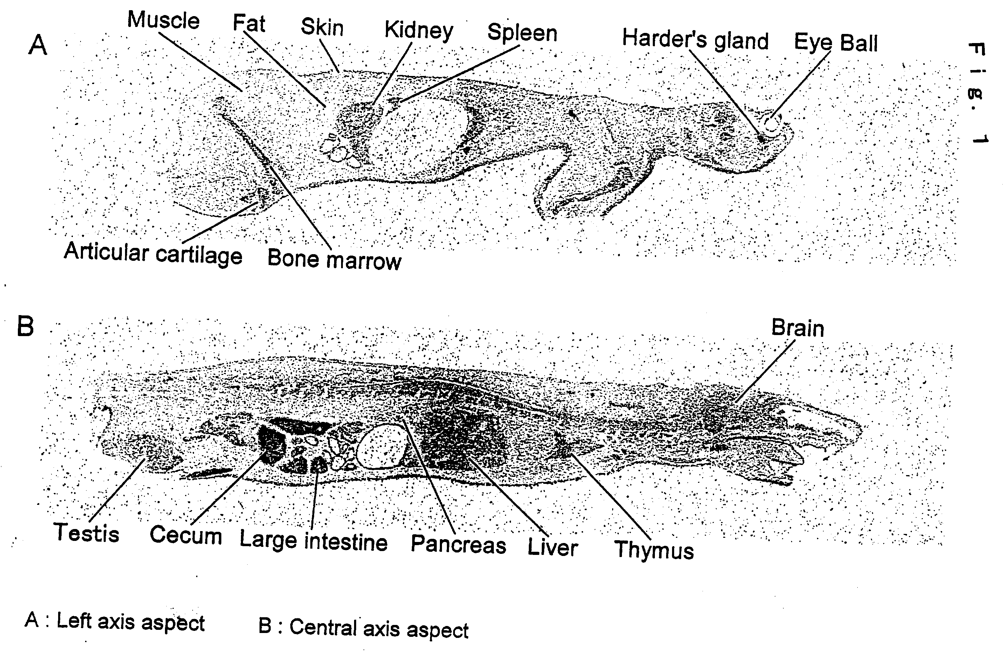

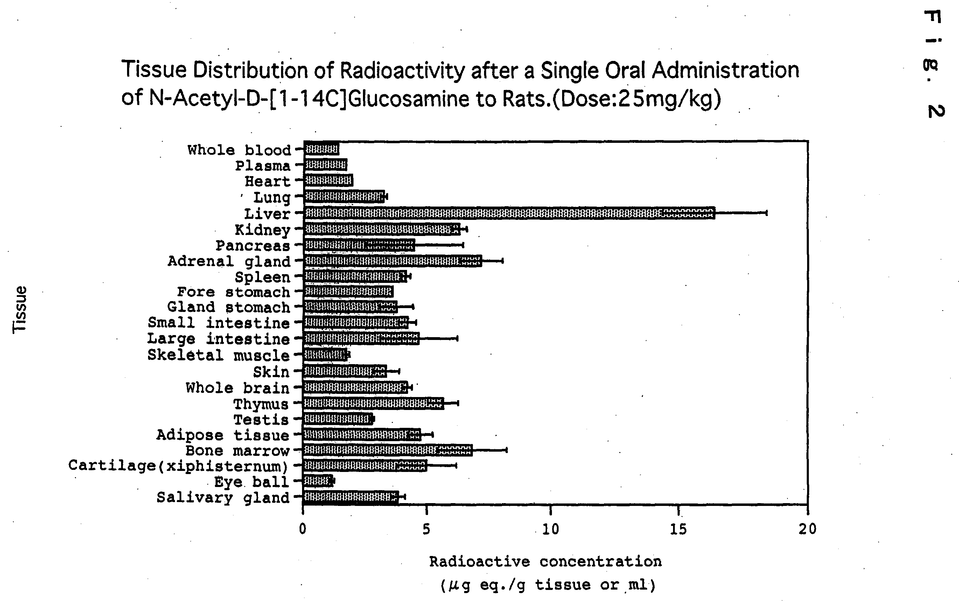

A mixture of $^{14}$C labelled NAG (1st-position carbon atom-labelled product: manufactured by Amersham Life Science Co.) and unlabelled NAG (the one prepared in PREPARATION EXAMPLE 1) was given to rats by single forced oral administration (250 mg/kg of body weight) for in vivo kinetics study. After the administration, the NAG was rapidly absorbed and the average concentration of radioactivity in the blood reached the maximum value 4 hours after the administration and showed prompt attenuation for 24 hours. About 60% of the administered NAG was utilized as an energy source and excreted as $CO_2$ to expiration. Further, about 20% was excreted in the urine and stools. With respect to the residual about 20 %, from the image analysis shown in the autoradiography as shown in Fig.1 and the results of radioactivity concentration analyses of respective tissues as shown in Fig.2, it was suggested that the residual about 20 % was widely transferred to e.g. cartilaginous tissues and fatty tissues in the living organism, and utilized as constituting substances of the living organism.

TEST EXAMPLE 3 (Influential test of NAG on skin)

Using hairless rats, the influence of orally administered NAG on the content of hyaluronic acid in skin was tested. The NAG prepared in PREPARATION EXAMPLE 1 was mixed in to a basic feed (Solid feed MF, manufactured by Oriental Kobo Kogyo Kabushiki Kaisha), and rats were permitted to freely ingest the mixture so that the substantial administered amount of NAG would be 0, 20 or 200 mg/kg of body weight/day. Administration was continued for 4 weeks from 9 week instar to 13 week instar, and the hyaluronic acid amounts in the skin layer were measured separately for epidermis and corium. In this connection, the measurement of the hyaluronic acid was carried out in accordance with a hyaluronic acid measurement kit (manufactured by Chugai Shindanyaku Co.). The results are shown in Table 1.

Table 1

| Ingested amount of NAG (mg/kg/day) | Hyaluronic acid content (µg/g dried tissue) | |
|---|---|---|
| | Epidermis | Corium |
| 0 | 31.25 | 462.7 |
| 20 | 33.77 | 506.6 |

Table 1 (continued)

| Ingested amount of NAG (mg/kg/day) | Hyaluronic acid content (μg/g dried tissue) | |
|---|---|---|
| | Epidermis | Corium |
| 200 | 35.09 | 549.8 |

As is evident from Table 1, it was confirmed that the hyaluronic acid contents in the epidermis and corium tend to increase together in proportion to the administered amounts of NAG.

TEST EXAMPLE 4 (Influential test of NAG on skin)

20 adult female volunteers of age ranging from 25 to 45 year old were classified into two groups i.e. a test group and a control group. They were asked to take tablets prepared similarly as in Example 1 as described below at a rate of 5 tablets per shot, twice a day (NAG ingestion amount per day: 1.2 g) together with water, and the tablets for the control group were prepared by using lactose as a placebo instead of NAG. The test period was 60 days, and after the completion of the test, they were questioned about skin conditions and the like by questionnaires. No particular restriction was made with respect to diet, makeup and the like during the test period. The results are shown in Table 2.

Table 2

| Items of questionnaires | Test region | Placebo region |
|---|---|---|
| Feel moistness in skin | 7 | 2 |
| Feel tension in skin | 6 | 2 |
| Fine wrinkles decreased | 5 | 1 |
| Skin conditions improved as a whole | 8 | 2 |
| Skin conditions worsened as a whole | 0 | 1 |
| No change (including unidentified) | 2 | 5 |

As is evident from Table 2, as compared with the placebo region, many persons of the test region felt that the skin conditions were improved as a whole, for example, feeling moistness or tension in the skin as compared with the conditions before the start of the test. Accordingly, the skin-beautification effect of NAG was recognized.

TEST EXAMPLE 5

To 22 females usually having a tendency of xeroderma and rough skin (average age: $25.5 \pm 10.7$), a double blind long-term ingestion study was carried out in the following manner wherein a placebo of non-NAG-containing tablets was given to a control group.

Subject group

NAG-containing tablets-administered group (NAG group, n=11): Tablets prepared similarly as in Example 1 as described below (NAG amount: 200 mg/tablet), were ingested at a rate of 5 tablets/day (NAG 1,000 mg/day, as a daily dose).

Placebo-administered group (Placebo group, n=11): Tablets prepared similarly wherein lactose was used instead of NAG, were ingested at a rate of 5 tablets/day.

Ingestion period and inspection period

The ingestion period was 8 weeks in each group. The inspection was in principle carried out just before the start of ingestion, 4 weeks after the start of ingestion, and just before the completion of ingestion (8 weeks after the start of ingestion).

Inspection process

(1) Dermatologic examination and doctor's questions

In the observation of the whole body, 4-ranked evaluation (0: no symptom, 1: slight, 2: medium, 3: severe) was conducted with respect to pruritus, desiccation, flushing, erosion, desquamation, papula, vesicle and tumefaction. In the observation of the face, 4-ranked evaluation was similarly conducted with respect to cosmetic dermatitis, desiccation, flushing and spread of cosmetics. Further, amelioration of general symptom including the observations of the whole body and face, was evaluated as a general observation. These evaluations were conducted by a plurality of doctors designated by the Japan Dermatologic Science Society. The results are shown in Table 3 (average value of each score of each group).

Table 3

| | | NAG group (n=11) | | |
|---|---|---|---|---|
| | | Number of symptomatic persons | Before ingestion | After 4 weeks | After 8 weeks |
| Face | Cosmetic dermatitis | 3 | 1.00 | 1.00 | 1.33 |
| | Dessication | 11 | 2.00 | 1.18** | 1.00* |
| | Flushing | 10 | 1.80 | 1.10* | 1.10* |
| | Spread of cosmetics | 6 | 1.83 | 1.33 | 0.83* |
| Whole body | Pruritus | 10 | 1.40 | 0.90 | 0.60 |
| | Dessication | 11 | 2.09 | 1.36* | 1.00* |
| | Flushing | 5 | 1.40 | 0.80 | 0.40 |
| | Erosion | 1 | 2.00 | 1.00 | 0.00 |
| | Desquamation | 3 | 1.33 | 1.33 | 1.00 |
| | Papula | 2 | 1.00 | 1.50 | 1.50 |
| | Vesicle | 1 | 1.00 | 2.00 | 2.00 |
| General observation | | 11 | 1.82 | 1.27* | 1.09* |
| | | Placebo group (n=11) | | |
| | | Number of symptomatic persons | Before ingestion | After 4 weeks | After 8 weeks |
| Face | Cosmetic dermatitis | 0 | - | - | - |
| | Dessication | 11 | 2.00 | 1.73 | 1.55 |
| | Flushing | 10 | 1.60 | 1.40 | 1.30 |
| | Spread of cosmetics | 6 | 2.17 | 1.50 | 1.67 |
| Whole body | Pruritus | 5 | 1.80 | 1.60 | 1.00 |
| | Dessication | 11 | 2.00 | 1.55* | 1.45* |
| | Flushing | 5 | 1.80 | 1.20 | 1.00 |
| | Erosion | 1 | 2.00 | 1.00 | 1.00 |
| | Desquamation | 2 | 1.00 | 1.00 | 1.00 |
| | Papula | 2 | 1.00 | 1.50 | 1.00 |
| | Vesicle | 1 | 1.00 | 1.00 | 2.00 |
| General observation | | 11 | 1.64 | 1.18 | 1.27 |

Wilcoxon test:
*:p<0.05,
**:p<0.01

As shown in Table 3, with respect to the symptoms of face, in the NAG group, effects for significant improvements were recognized in the items of "dessication", "flushing" and "spread of cosmetics". On the other hand, in the placebo group, no significance was recognized in any item of observation. Further, in the symptom of whole body, significant improvements were recognized in the item of "dessication" in both groups. In the general observation, although significant improvements were recognized in the NAG group, no significant improvement was recognized in the placebo group.

(2) Moisture content, Oil and fat content, and Acidity (pH)

The moisture content was measured by using Corneometer CM825 (manufactured by Courage+Khazaka Electronic Gmbh). This apparatus measures the moisture content of the epidermis by measuring the electrostatic capacity via corneal layer, and is known to be more accurate than conventional impedance methods or infrared spectrophotometric methods.

The oil and fat content was measured by using Sebumeter SM810 (manufactured by Courage+Khazaka Electronic GmbH). In the use of this apparatus, a special tape which absorbs only the oil and fat content is attached to the measurement site for 30 seconds and the oil and fat content is measured by the change of light transmittance of the tape.. This apparatus is known not to be affected by humidity, etc.

The acidity was measured with PH 900 (manufactured by Courage+Khazaka Electronic Gmbh). In the use of this apparatus, an electrode is contacted to a skin surface through an ion-permeable membrane adjacent to a glass membrane and the acidity can be measured without electrochemical invasion. In this connection, the optimum pH of female skin is about 5.5.

Measurement sites were 1 cm below the left eye, the medial part of the left upper arm (3 cm above the elbow), and the poll (3 cm below the spinal pivot of the neck). With respect to the oil and fat content, since most subjects were scored at 0 at the left upper arm and the poll, only the site below the left eye was measured from the 2nd inspection.

In order to keep the environments for the measurements as equal as possible, a room having its internal conditions adjusted to be constant was prepared before the measurements (room temperature: 18 to 20°C, humidity: 45 to 60 %), and the subjects were asked to remain at rest in this room for at least 30 minutes. Further, with respect to the makeup at the measurement site, it was in principle prohibited to put on makeup from 60 minutes before the inspection. The results are shown in Table 4.

Table 4

| | | NAG group (n=11) | | |
| --- | --- | --- | --- | --- |
| | | Before ingestion | After 4 weeks | After 8 weeks |
| Moisture content | Below left eye | 48.0±8.8 | 58.8±14.2* | 56.2±8.4** |
| | Left upper arm | 37.8±7.8 | 38.7±5.8 | 36.2±6.8 |
| | Poll | 51.3±5.6 | 51.9±5.4 | 52.1±10.9 |
| Acidity (pH) | Below left eye | 6.0±1.0 | 5.7±0.5 | 5.8±0.4 |
| | Left upper arm | 5.5±0.3 | 5.6±0.3 | 5.7±0.4 |
| | Poll | 5.7±1.1 | 5.4±0.3 | 5.3±0.4 |
| Oil and fat content | Below left eye | 63.8±42.3 | 53.0±29.2 | 40.8±18.7* |
| | | Placebo group (n=11) | | |
| | | Before ingestion | After 4 weeks | After 8 weeks |
| Moisture content | Below left eye | 58.6±11.7 | 57.8±10.4 | 48.1±10.2* |
| | Left upper arm | 37.6±9.5 | 36.5±6.7 | 32.2±8.5 |
| | Poll | 51.7±18.9 | 49.2±14.1 | 53.6±20.8 |
| Acidity (pH) | Below left eye | 5.7±0.6 | 5.7±0.5 | 5.8±0.5 |
| | Left upper arm | 5.4±0.4 | 5.7±0.4* | 5.6±0.5 |
| | Poll | 5.7±0.7 | 5.5±0.4 | 5.6±0.4 |
| Oil and fat content | Below left eye | 37.1±32.2 | 30.1±20.3 | 42.6±30.1 |

Wilcoxon test:
*: $p<0.05$,
**: $p<0.01$

As shown in Table 4, a significant increase was confirmed in the moisture content at the site below the left eye in the NAG group. Further, a significant decrease in the oil and fat content was confirmed. On the other hand, in the placebo group, a significant decrease was recognized in the moisture content at the site below the left eye.

(3) Analysis by a microscopic three-dimensional skin surface analyzer (VISIONSCAN)

This analysis was conducted by using a digital analyzer of the skin surface (VISIONSCAN: manufactured by Courage+Khazaka Electronic Gmbh). In the use of this apparatus, the skin surface was irradiated with a special ultraviolet ray source, and the image is taken by a high performance CCD camera and digitalized for evaluation. The following factors were sampled as parameters.

(a) SEsm (Skin Smoothness)

This value is calculated from the average of the width and depth of wrinkles by the following formula (i), and is one of the indices which show the smoothness of skin. The smaller this value is, the smoother the skin surface is.

$$SEsm = (Co-Cu)*(Fmx-Fmy)*K \tag{i}$$

Fmx: average width of furrows for the row analysis.
Fmy: average width of furrows for the column analysis.
Co: right frontier of the histogram whose calculation is based on reference values.
Cu: left frontier of the histogram whose calculation is based on reference values.
K: factor

(b) SEr (Skin roughness)

This value is obtained by calculating the ratio of points darker than the reference points in the whole image and further calculating by the following formula (ii), and is one of the indices which show the roughness of skin. The higher the value is, the rougher the skin is.

$$SEr = I/(Nx*Ny)*100 \qquad (ii)$$

I: counter whose start value is 0 and which is incremented each time the gray value of the current point is smaller than the threshold issued from the set-up programs.
Nx: amount of points per row.
Ny: amount of points per column.

(c) SEsc (Skin Scaliness)

Epidermolysis parts are counted to be brighter than the reference values in the image. The SEsc value is obtained by calculating the ratio of these parts relative to the entire part by the following formula (iii), and is one of the indices which show the dryness of the scale (corneum). The lower the value is, the more moist and the less epidermolysis (scale).

$$SEsc = I/(Nx*Ny)*100 \qquad (iii)$$

I: counter whose start value is 0 and which is incremented each time the gray value of the current point is bigger than the threshold issued from the set-up programs.
Nx: amount of point per row.
Ny: amount of point per column.

(d) SEw (Skin Wrinkles)

This value is an index which is calculated by the following formula (iv) and shows the surface texture in vertical and horizontal directions of skin or the number and width of wrinkles. The higher the value is, the more the number of wrinkles is and the wider the width of the wrinkles is.

$$SEw = Fmx*Fmy/(Fax*Fay)*Fay/Fax*K \qquad (iv)$$

Fax: amount of furrows for the row analysis.
Fmx: average width of furrows for the row analysis.
Fay: amount of furrows for the column analysis.
Fmy: average width of furrows for the column analysis.
K: factor

(e) Correction K (Kurtosis)

This value shows the smoothness of the whole skin. This value shows the quality of histogram in the hue point of skin. The closer to 0 the value is, the smoother in curve the hue point is and the closer to ideal skin. The results of the above tests (a) to (e) are shown in Table 5.

Table 5

| | | NAG group (n=11) | | |
|---|---|---|---|---|
| | | Before ingestion | After 4 weeks | After 8 weeks |
| Kurtosis (Ideal value:0) | Below left eye | 0.58 | 0.34 | 0.30 |
| | Left upper art | 0.97 | 0.40 | 0.50 |
| | Poll | 0.79 | 0.39 | 0.39 |
| SEsm (Ideal value: Lowest value) | Below left eye | 378.1 | 337.6 | 309.4 |
| | Left upper art | 384.7 | 324.7 | 379.9 |
| | Poll | 443.8 | 338.9* | 345.2* |
| SEr (Ideal value: Lowest value) | Below left eye | 0.25 | 0.26 | 0.21 |
| | Left upper art | 0.30 | 0.22 | 0.28 |
| | Poll | 0.51 | 0.32 | 0.40 |
| SEsc (Ideal value: Lowest value) | Below left eye | 238.4 | 137.0 | 133.0 |
| | Left upper art | 342.9 | 195.8 | 202.4 |
| | Poll | 352.4 | 201.6 | 169.3* |
| SEw (Ideal value: Lowest value) | Below left eye | 33.2 | 29.0 | 27.8 |
| | Left upper art | 30.2 | 26.4 | 31.9 |
| | Poll | 27.3 | 23.4 | 29.5 |
| | | Placebo group (n=11) | | |
| | | Before ingestion | After 4 weeks | After 8 weeks |
| Kurtosis (Ideal value:0) | Below left eye | 0.42 | 0.76* | 0.30 |
| | Left upper art | 0.43 | 0.59 | 0.42 |
| | Poll | 0.58 | 0.53 | 0.43 |
| SEsm (Ideal value: Lowest value) | Below left eye | 379.6 | 398.8 | 353.7 |
| | Left upper art | 335.7 | 373.1 | 335.1 |
| | Poll | 387.2 | 414.1 | 366.2 |
| SEr (Ideal value: Lowest value) | Below left eye | 0.24 | 0.23 | 0.24 |
| | Left upper art | 0.10 | 2.51 | 0.22 |
| | Poll | 0.35 | 0.33 | 0.44 |
| SEsc (Ideal value: Lowest value) | Below left eye | 166.8 | 146.7 | 158.0 |
| | Left upper art | 214.1 | 256.1 | 246.5 |
| | Poll | 231.8 | 219.7 | 202.2 |
| SEw (Ideal value: Lowest value) | Below left eye | 29.8 | 33.6 | 33.7 |
| | Left upper art | 23.7 | 28.1 | 24.4 |
| | Poll | 27.5 | 26.0 | 29.3 |

Wilcoxon test:
  *: $p < 0.05$

As shown in Table 5, in the NAG group, a significant decrease was confirmed in the SEsm value and the SEsc value at the poll, whereby it was found that the smoothness of skin was recovered, the dryness of corneum was reduced and the scale was decreased. On the other hand, in the placebo group, no significant decrease was confirmed in any value.

EXAMPLE 1

Respective materials as indicated in Table 6 were mixed and granulated by a fluidized bed granulator, and then triangular tablets of 300 mg/tablet were formed by a tabletting machine (NAG content: 120 mg/tablet). The tabletting property was excellent.

Table 6

| NAG | 40 wt% |
| Collagen | 30 wt% |
| Lactose | 15 wt% |
| Cellulose powder | 10 wt% |
| Citric acid | 2 wt% |
| Perfume | 2 wt% |
| Sucrose fatty ester | 1 wt% |
| Total | 100 wt% |

EXAMPLE 2

Respective materials were kneaded so that the incorporated amounts per capsule (300 mg/capsule) would be as indicated in the following Table 7, and triangular soft capsules were prepared by a soft capsule filling machine by using gelatin as an encapsulating agent. The filling property was excellent.

Table 7

| NAG | 30 mg |
| Vitamin E | 50 mg |
| Soybean lecithin | 20 mg |
| Safflower oil | 170 mg |
| Vitamin C | 20 mg |
| Glycerol fatty ester | 5 mg |
| Beeswax | 5 mg |
| Total | 300 mg/tablet |

EXAMPLE 3

Respective materials were mixed in the proportion as indicated in Table 8 and granulation was carried out using a 0.5 % guar gum solution as a binder by a fluidized bed granulator, to obtain 9.7 kg of NAG-containing granules. No moisture absorption of NAG was observed, dispersion of powder was good, and uniform granules were prepared.

Table 8

| NAG | 1.5 kg |
| Calcinated cow bone powder | 0.8 kg |
| Chondroitin sulfate | 0.3 kg |

Table 8 (continued)

| | |
|---|---|
| Vitamin C | 0.1 kg |
| Vitamin B mixture | 0.1 kg |
| Glucose | 2.1 kg |
| Dextrin | 3.5 kg |
| Citric acid | 0.4 kg |
| Lemon fruit juice powder | 1.2 kg |
| Total | 10.0 kg |

EXAMPLE 4

All of respective materials were dissolved in water in the proportion as indicated in the following Table 9 to prepare a paste-like sample.

Table 9

| | |
|---|---|
| NAG | 30 g |
| Blue berry extract | 100 g |
| Citric acid | 10 g |
| White refined sugar | 50 g |
| Pectin | 1 g |
| Perfume | 0.2 g |
| Water | 109 g |
| Total | 300.2 g |

This product was stable even after cold storage for one year.

EXAMPLE 5 (Candy)

Candy was prepared in accordance with a conventional method in the proportions indicated in the following Table 10.

This candy could be prepared by usual steps without NAG causing browning.

Table 10

| | |
|---|---|
| NAG | 20 wt% |
| Sugar | 36 wt% |
| Starch syrup | 40 wt% |
| Fruit juice | 3 wt% |
| Acidifier | 0.5 wt% |
| Coloring matter, Perfume | 0.5 wt% |
| Total | 100 wt% |

EXAMPLE 6 (Gummi)

Gummi was prepared in accordance with a conventional method in the proportions indicated in the following Table 11. The production of gummi includes a step of heating for evaporation of water after a step of mixing respective starting materials. Accordingly, in Table 11, the finished amount is less than the total of respective materials.

Table 11

| NAG | 200 g |
|---|---|
| Granulated sugar | 170 g |
| Starch syrup | 260 g |
| Sorbitol | 180 g |
| Citric acid | 2 g |
| Coloring matter, Perfume | 0.5 g |
| Gelling agent | 80 g |
| Water | 200 g |
| Finished amount | 1,000 g |

EXAMPLE 7 (Cookie)

A cookie was prepared in accordance with a conventional method in the proportions indicated in the following Table 12.

This cookie could be prepared by usual steps without NAG causing browning.

Table 12

| NAG | 80 g |
|---|---|
| Unsalted butter | 120 g |
| Sugar | 60 g |
| Egg | 20 g |
| Weak flour | 180 g |
| Baking powder | 1 g |
| Cocoa | 20 g |
| Milk | 10 g |

EXAMPLE 8 (Jelly)

Jelly was prepared in accordance with a conventional method in the proportions indicated in the following Table 13. The production of jelly includes a step of heating for evaporation of water after a step of mixing respective starting materials. Accordingly, in Table 13, the finished amount is less than the water amount mixed with the starting materials.

Table 13

| NAG | 100 g |
|---|---|
| Gelling agent | 3.5 g |
| Sugar | 50 g |
| Fruit juice | 10 g |
| Perfume, Coloring matter | Proper quantity |
| Acidifier, Sweetener | Proper quantity |

Table 13 (continued)

| Water | 1,000 ml |
|---|---|
| Finished amount | 750 ml |

EXAMPLE 9 (Powdered soup)

Powdered soup was prepared in accordance with a conventional method in the proportions indicated in the following Table 14.

This powdered soup could be dissolved in hot water readily, and its taste was good.

Table 14

| NAG | 4 g |
|---|---|
| Chicken consomme | 0.5 g |
| Dehydrated seaweed (Wakame) | 0.4 g |
| Sesame oil | 0.1 g |
| | 150 ml of hot water/meal |

EXAMPLE 10 (Refreshing drink)

A refreshing drink was prepared in accordance with a conventional method in the proportions indicated in the following Table 15.

Table 15

| NAG | 1,000 mg |
|---|---|
| Collagen | 100 mg |
| Ca lactate | 1,000 mg |
| $MgCl_2$ | 50 mg |
| Mixture of vitamins | 60 mg |
| Acidifier, Perfume | Proper quantity |
| Sucrose, Glucose, Liquid sugar | Proper quantity |
| Preserver | Proper quantity |
| | 50 ml /bottle |

As described above, according to the present invention, it is possible to obtain a skin care agent by which rough skin and fine wrinkles can be prevented or ameliorated. By orally ingesting the skin care agent of the present invention, NAG is rapidly absorbed and transferred to skin layer, and then becomes a starting material of hyaluronic acid or the like, by which the moisture and tension of skin can be improved and the rough skin and fine wrinkles can be prevented or ameliorated.

**Claims**

1.    A skin care agent which comprises N-acetylglucosamine as an active ingredient.

2.    A skin care agent according to claim 1 which further comprises at least one component selected from collagen, chondroitin sulfate, hyaluronic acid, vitamin C, a vitamin of the B group, trehalose and ceramide.

3.    A skin care agent according to claim 1 which further comprises at least one component selected from collagen, chondroitin sulfate and vitamin C.

4. A skin care agent according to any preceding claim wherein the N-acetylglucosamine is present in an amount of at least 0.1% by weight.

5. A skin care agent according to any preceding claim wherein the N-acetylglucosamine is present in an amount of from 1% to 50% by weight.

6. A skin care agent according to any preceding claim in the form of tablets, capsules, powder, granules, liquid or paste.

7. Use of a skin care agent according to any preceding claim as a cosmetic product.

8. A method of cosmetic treatment of the human body, comprising the oral administration of N-acetylglucosamine to said body in an amount of from 0.1 g to 15 g per day.

9. A method according to claim 8 wherein the amount of N-acetylglucosamine administered to said body is from 0.3 g to 5 g per day.

10. A health food or beauty care food which comprises a skin care agent according to any of claims 1 to 6.

# Fig. 1

A

Muscle  Fat  Skin  Kidney  Spleen  Harder's gland  Eye Ball

Articular cartilage  Bone marrow

B

Brain

Thymus

Liver

Pancreas

Large intestine

Cecum

Testis

A : Left axis aspect   B : Central axis aspect

F i g. 2

Tissue Distribution of Radioactivity after a Single Oral Administration of N-Acetyl-D-[1-14C]Glucosamine to Rats.(Dose:25mg/kg)